Europäisches Patentamt

**European Patent Office**

**Office européen des brevets**

(19)

(11) Publication number: **0 123 474**
**A2**

(12) # EUROPEAN PATENT APPLICATION

(21) Application number: **84302417.5**

(22) Date of filing: **09.04.84**

(51) Int. Cl.³: **A 61 G 1/00**

(30) Priority: **20.04.83 US 486567**

(43) Date of publication of application:
**31.10.84 Bulletin 84/44**

(84) Designated Contracting States:
**AT BE CH DE FR GB IT LI LU NL SE**

(71) Applicant: **PACKAGING CORPORATION OF AMERICA**
**1603 Orrington Avenue**
**Evanston Illinois 60204(US)**

(72) Inventor: **Nederveld, Terriel L.**
**3113 Howlett Drive S.E.**
**Ada Michigan 49301(US)**

(74) Representative: **Wilson, Joseph Martin et al,**
**WITHERS & ROGERS 4 Dyer's Buildings Holborn**
**London EC1N 2JT(GB)**

(54) **A patient carrier.**

(57) A patient carrier is provided which is formed of multi-ply fibreboard material. The carrier includes an elongated, substantially rigid, planar panel which is adapted to supportingly engage a patient. Means are provided along opposing elongated sides of the panel for accommodating elongated pliable elements which partially encompass the patient and retain same on the panel while being transported on the carrier.

EP 0 123 474 A2

Croydon Printing Company Ltd.

# A PATIENT CARRIER

## Background of the Invention

In the transporting of patients such as accident victims in emergency vehicles such as paramedic ambulances or vans, it is the practice to initially place the patient on either a stiff carrier or a collapsible stretcher. The stiff carrier is preferred because the patient may remain on the carrier while being transported by the paramedics or emergency personnel, to the hospital, clinic, or doctor's office; while the patient is being moved within the hospital, clinic, or doctor's office; and while the patient is undergoing surgery, tests, and the like and thus, reduce significantly the possibility of further injury or pain to the patient caused by being moved from the initial stretcher or carrier onto various other conveyances, beds, tables, and the like. In most paramedic vehicles, a stiff carrier is customarily used which is formed from relatively thick (e.g., 3/4") plywood or like material. Such a carrier is expensive (e.g., $125.00/carrier) and for reasons of economy must be reusable. Heretofore it has been the practice for a patient to be retained on the carrier by a plurality of expensive buckle-type straps which might require a substantial amount of time and dexterity to effect removal of the straps from around the patient. Because of the need to reuse the carrier, the paramedic personnel are oftentimes subjected to prolonged periods of delay awaiting the return

- 2 -

0123474

of the carrier from the hospital, clinic, or doctor's office. Furthermore, before the carrier and associated straps can be reused, they frequently must be thoroughly cleaned.

Because of the aforenoted shortcomings besetting such carriers, most paramedic units cannot afford to stock a large number of such carriers to cope with possible disasters or emergencies wherein an inordinate number of victims are involved requiring immediate medical attention and removal from the scene of the disaster or emergency. The size and weight of a plywood carrier oftentimes make it difficult and awkward to manually manipulate the carrier to the location of the victim (e.g., pinned within a vehicle interior). Thus, in such a situation the victim must frequently be manually moved a substantial distance in substantially unsupported condition before being placed on the carrier with the result that the victim suffers serious life-threatening consequences. In some instances, because the patient-supporting surface of the carrier is smooth, slippery, and planar, it is difficult to properly retain the victim on the carrier while being manipulated down stairwells, ladders, and the like, even though various restraining devices are utilized. As aforementioned, typical restraining devices used for this purpose are buckle-type straps wherein the strap webbing is susceptible to becoming fouled or the buckle becoming jammed or broken.

## Summary of the Invention

Thus, it is an object of the invention to provide an improved carrier which avoids all of the aforementioned shortcomings associated with prior stretchers or carriers of this general type.

It is a further object to provide an improved carrier which is sturdy, lightweight, of minimal cost, and may be readily expended without incurring a substantial financial expense once the patient has been properly attended to and thus, significantly improve the utilization of the paramedic and/or emergency personnel's time.

It is a further object to provide an improved carrier having elongated foldable side sections which coact with an elongated center section and the patient-engaging elements to securely accommodate and retain the patient on the carrier while being transported thereby.

It is a still further object to provide an improved carrier which is resistant to moisture, blood, petroleum products and the like.

It is a still further object to provide an improved carrier which provides effective support for the back, neck, and head of the patient while being extricated from a confined space such as the interior of an automobile, truck cab, or the like which was involved in an accident or disaster.

It is a still further object to provide an improved carrier which retains its strength and utility even when immersed in water.

Further and additional objects will appear from the description, accompanying drawings, and appended claims.

In accordance with one embodiment of the invention a patient carrier is provided which is formed from a sheet of multi-ply fibreboard material. The carrier includes an elongated substantially rigid, planar panel which supportingly engages the backside of a patient. Disposed along the elongated opposed sides of the planar panel are means for accommodating elongated pliable patient engaging elements which embrace the patient and retain same on the panel while being transported by the carrier.

For a more complete understanding of the invention, reference should be made to the drawings wherein:

Fig. 1 is a fragmentary perspective top view of one form of the improved patient carrier.

Fig. 2 is a top plan view of one of the components of the carrier shown in Fig. 1 and showing in phantom lines an end panel foldably connected to one end of a center panel forming the component in question.

Fig. 3 is a top plan view of a second form of the improved patient carrier.

Fig. 4 is a perspective view similar to Fig. 1 but showing a patient accommodated on the carrier.

Fig. 5 is a fragmentary enlarged view of the carrier of Fig. 4 and showing how the legs of the patient are held in place on the carrier.

Fig. 6 is a perspective view showing how the carrier of Fig. 4 is being manually maneuvered down a stairwell.

Figs. 7 and 8 are perspective views of a patient being secured to the carrier of Fig. 3.

Figs. 9 and 10 are perspective views showing the patient of Fig. 8 being transferred onto the carrier of Fig. 1.

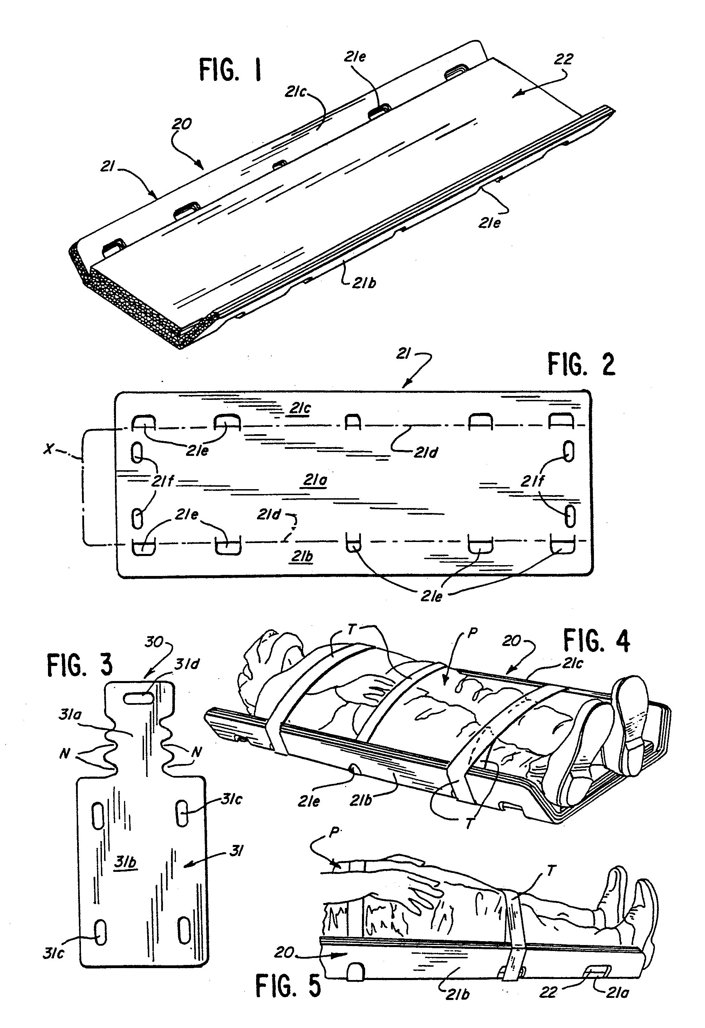

Referring now to drawings and more particularly to Figs. 1, 2, 4, and 5, one form of an improved patient carrier 20 is shown. Carrier 20 has particular utility for use by paramedic and emergency personnel for transporting victims or stricken persons from the scene of a disaster, accident, or an emergency situation to a hospital, clinic, or doctor's office in an expeditious and facile manner. Carrier 20 is preferably formed of a multi-ply fibreboard material, e.g., a laminated triple wall corrugated board, which is sturdy, relatively lightweight, and inexpensive.

Carrier 20 includes a first section 21 and a second section 22, both formed of the same material. Section 21, as seen more clearly in Fig. 2, includes an elongated center panel 21a (e.g., 72" x 17") and a pair of side panels 21b, 21c which are connected by foldlines 21d to the opposite elongated side edges of center panel 21a. Each foldline 21d is interrupted by a plurality of longitudinally spaced openings 21e. The function of the openings 21e will be described more fully hereinafter. A pair of laterally spaced handholes 21f are formed in center panel 21a adjacent each narrow end of panel 21a. If desired, an end panel X, shown in phantom lines in Fig. 2, may be foldably connected to at least one end edge of the center panel 21a.

The second section 22 comprises a single panel which is secured by suitable adhesive in substantially coincident relation to the upper surface of center panel 21a, see Fig. 1. Section 22 provides a cushion pad for the patient P when disposed in a prone position on the carrier, see Fig. 4. Where the carrier is formed of laminated triple-wall corrugated material or multi-plies of double wall corrugated material, the corrugations thereof should extend longitudinally of panels 21a and 22 thereby providing maximum strength for the carrier. The corrugations of side panels 21b, 21c should also extend longitudinally thereof. Once the patient P has been placed in a prone position (normally face up) on the exposed surface of panel 22, the side panels 21b, 21c and the end panel X, if there is one, are folded to upright positions so that the patient is accommodated therebetween. Preferably the end panel X is disposed adjacent the feet of the patient. If the height of the patient is greater than the length of the carrier 20, obviously the end panel X would remain in an unfolded position. The openings 21e formed along the elongated sides of the panel 21a serve a dual function: a) they allow sections of reinforced self-bonding tape or the like to pass therethrough and substantially embrace and fixedly retain the patient in proper position on the exposed surface of panel 22; and b) they serve as drain-holes and handholes to facilitate manually carrying by two or four persons the loaded carrier. Where only two persons are carrying the loaded carrier, normally one person $M_1$, is at the head of the carrier and the other person $M_2$ is disposed at the foot end of the carrier and the handholes 21f at the end of panel 21a, accessible from the underside of the carrier, may be utilized. The patient P preferably remains on the carrier while in the hospital, clinic, or doctor's office until the testing (e.g., X-ray), emergency surgery, or other necessary treatment for the patient has been

- 6 -                                        0123474

completed. Thus, by reason of having the patient remain on the carrier during such procedures, the patient is more comfortable and less susceptible to life-threatening or serious injury which might otherwise result due to the patient being removed repeatedly, while in an unsupported condition, from the carrier, various other conveyances, tables, and/or beds.

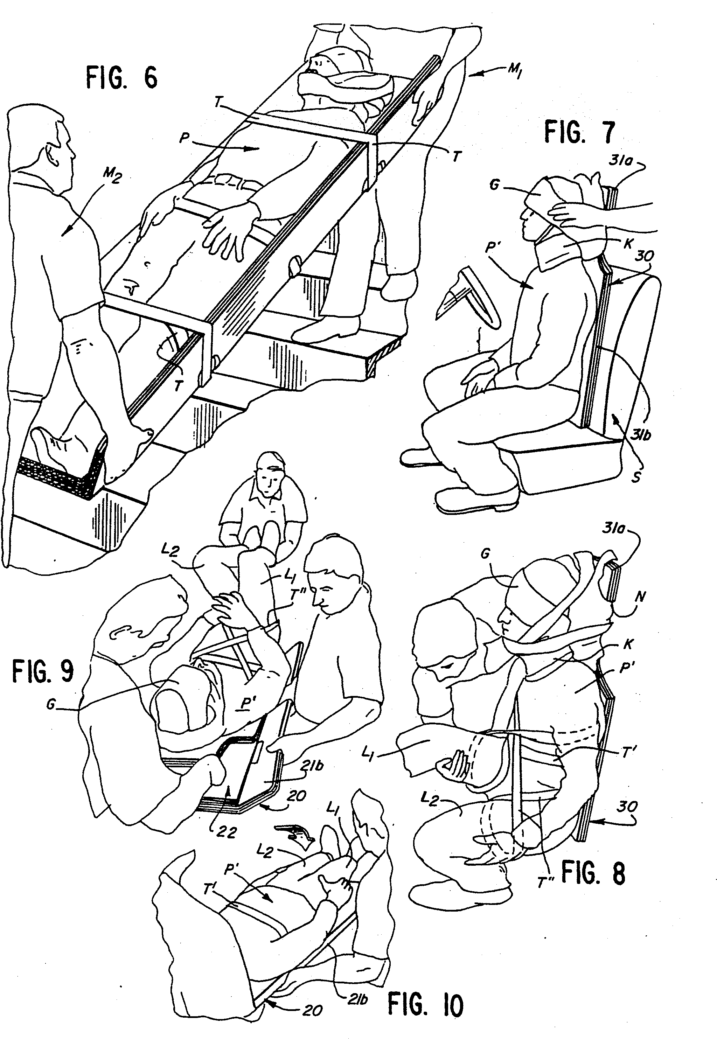

When the patient is undergoing tests, surgery, or the like, one or more of the tapes may be readily cut and removed if necessary. Because the tape T is preferably of a thin reinforced plastic X-ray tested web-like material and self-bonding, no buckles, snaps, or other fasteners need to be manipulated by the attending doctor, technician, or their aides but the tape merely cut with a scissor or knife in order to remove same from selected areas of the carrier and supported patient. While reinforced, self-bonding, plastic tape T is preferred because of its low cost, ease of handling, light weight and strength, other types of securing means such as leather straps and belts, cloth strips, cloth belts and the like may also be utilized with the improved carrier, if desired. Whatever type of patient-securing element is utilized, it normally engages the underside of the center panel 21a and the opposite ends are inserted through the selected openings 21e and then drawn tightly over the portion of the patient aligned between the selected openings. Additional tapes, straps, strips, etc. may be utilized which not only engage the underside of the center panel, but the outer surfaces of the upright side panels 21b, 21c, as well and thus, firmly hold the side panels against the adjacent sides of the accommodated patient, see Figs. 4 and 6.

A second form of carrier 30, shown in Fig. 3, is intended for use in facilitating extricating a victim P' from a confined location, such as a driver or passenger being pinned within the interior of an automobile or truck cab. As in the case of carrier 20, carrier 30 is preferably formed from a sheet of laminated triple wall corrugated fibreboard. Carrier 30 includes an elongated panel 31 which has a substantially shorter length (e.g., 35") than the center panel 21a of carrier 20. The reason for the shorter length is that the panel 31 is intended to support only the back, neck, and head of the patient P' while the latter is being extricated from the confined location. The upper portion 31a of panel 31 is relatively narrow and the neck and head of the accommodated patient P' is intended to be aligned therewith after a cervical collar K has been applied to the neck of the victim. The opposed side edges of the upper portion 31a may be provided with a plurality of notches N which are adapted to accommodate segments of the gauze, tape, and similar types of dressings G utilized in covering the head or neck wounds which the patient might have suffered. As seen in Fig. 8, the head of the patient is secured with the necessary gauze and tape G by the paramedic or emergency personnel after the carrier 30 has been inserted between the back of the patient and the back of the seat S of vehicle in which the patient was riding at the time of accident. The torso of the patient is then secured to the lower portion 31b of the panel 31 by suitable tape T' so as to prevent spinal movement of the victim. Suitable openings 31c are provided along the sides of lower portion 31b and are adapted to accommodate segments of the tapes T' or belts, strips, etc., which are utilized to retain the patient's torso against the panel lower portion 31b. The tape T' is applied to the carrier 30 and patient P' by a paramedic or emergency personnel in the same manner as described with regard to carrier 20. Where possible the legs $L_1$, $L_2$ of the patient are retained in a bent position, as seen in Fig. 8, by the tape T" or strap being passed under the thigh of one leg $L_1$, through selected openings 31c, and then around behind the panel lower portion 31b. A similar procedure may be followed for supporting the other leg $L_2$ in a

similar bent position. The procedure followed with regard to supporting the legs in a bent position will depend upon the relative position of the patient within the confined location of the automobile interior, truck cab, or the like at the time of the accident.

After the head, neck, torso, and legs of the patient have been properly secured to the carrier 30 in the manner as aforedescribed, the patient P' and attached carrier 30 are manually moved simultaneously as a unit from the confined location and then carefully placed on the exposed surface of the pad section 22 of the larger carrier 20, see Fig. 9. The tapes T" holding the patient's legs in a bent position are then released allowing the legs to be straightened out and secured to carrier 20 in a manner as shown in Fig. 5. During this manipulation onto carrier 20, the carrier 30 remains attached to the head, neck, and torso of the patient until the latter has undergone the necessary tests, surgery, or required treatment. The maximum width of the carrier panel 3I is slightly less than the maximum width of the center panel 21a of carrier 20. Thus, the side panels 21b, 21c of carrier 20 may be folded to upright positions after the carrier 30 and attached patient P' have been properly spotted on the pad panel 22 of carrier 20.

As observed in Fig. 3, the narrow upper portion 31a of the carrier panel 31 is provided with a handhole 31d which facilitates manual manipulation of the carrier 30 between the patient P' and the back of the seat S on which the victim is disposed, see Fig. 7. Also, handhole 31d facilitates pulling the carrier and attached patient from the confined location.

As aforementioned, both carriers 20 and 30 are formed from laminated triple-wall corrugated fibreboard or laminated double-wall corrugated fibreboard or other similar material. In either case the fibreboard or other material is made resistant to water, blood, petroleum products, and the like by various methods (e.g., cascading) well known in the art. By reason of the moisture resistance of the fibreboard material, the improved carriers 20, 30 may be used underwater in emergencies involving boat accidents, floods, etc., without deleteriously affecting the strength of the carrier. Where the carrier is to be used under such an environment, it may be desirable to secure the tapes, etc., to the underside or backside of the carrier before attempting to position the carrier into engagement with the victim.

The carriers 20 and 30 may be colored in distinctive colors so that they may be readily located when necessary. Furthermore, indicia may be imprinted on the exposed surfaces of the carriers 20, 30 setting forth instructions on how the carrier is to be used and how the patient is to be placed thereon. The fibreboard material enables tags or labels to be readily stapled or otherwise secured to areas of the carrier identifying the accommodated patient, a diagnosis of the patient's condition, and what medical treatment should be administered.

The number, location, size, and shape of the openings 21e, 21f, 31c, and 31d, as well as the size and shape of the carriers themselves, may be varied from that shown without departing from the scope of the invention. Certain of the openings may be utilized to snugly receive poles, standards, or the like on which containers, such as bottles, bags, or the like may be mounted when intravenous solutions are to be administered to the patient while being transported on the carrier. In addition, other emergency devices such as those commonly referred to as "heart thumpers" may be utilized while the patient is secured to the carrier. Because the carriers 20, 30 are substantially planar, a large number thereof may be stored or shipped while in a stacked relation.

Thus, it will be noted that an improved patient carrier has been provided which is of simple, inexpensive, lightweight, yet strong, construction; has great versatility; is easy to manipulate; and enables the patient to be securely held in place on the carrier while the latter is being manipulated down or up stairwells, ladders, and the like. Because of the relatively small cost of the improved carrier and the versatility thereof, a greater distribution and utilization of such carriers by the police, fire and civil defense units, paramedics, first aid groups, ski patrols, and various other organizations can be attained without incurring inordinate or excessive costs.

1. A patient carrier formed of multi-ply fibreboard sheet material comprising an elongated, substantially rigid, planar panel for supportingly engaging the backside of a patient; and means formed along elongated opposed sides of said panel for accommodating elongated pliable patient engaging elements and retaining the patient on the panel while being transported by said carrier.

2. The carrier of claim 1 wherein the sheet material includes triple wall corrugated fibreboard with the corrugations thereof extending substantially longitudinally of the elongated panel.

3. The carrier of claim 1 wherein the elongated panel includes elongated side sections foldably connected to opposed peripheral segments of an elongated patient-supporting center section and movable between operative and inoperative modes; when in an operative mode, said side sections extending angularly upwardly from said center section and being adapted to accommodate therebetween the patient supported by said center section, and when in an inoperative mode, said side sections being in a non-folded relation with said center section.

4. The carrier of claim 3 wherein the means for accommodating the patient engaging elements are disposed adjacent to the folding connections between the center section and the side sections.

5. The carrier of claim 1 wherein the means for accommodating the patient engaging elements include a plurality of longitudinally spaced openings disposed adjacent to the opposed sides of said panel.

6. The carrier of claim 1 wherein the means for accommodating the patient engaging elements includes a plurality of longitudinally spaced recesses formed in the opposed peripheral sides of said panel.

7.   The carrier of claim 1 wherein a finger opening is formed in at least one end portion of the elongated panel.

8.   The carrier of claim 1 wherein one end portion of the elongated panel is narrow relative to the remainder of the panel and is adapted to supportingly engage the head of the patient.

9.   The carrier of claim 8 wherein opposed peripheral sides of the panel one end portion are provided with means for accommodating the patient engaging elements.

10.   The carrier of claim 9 wherein the panel narrow one end portion is provided with a finger opening.

11.   A carrier formed of multi-ply fibreboard sheet material for transporting a patient while in a substantially prone position, said carrier comprising a first panel having an elongated substantially planar rigid center section for subtending the patient and elongated side sections integral with opposed elongated peripheral segments of said center section, and means adjacent the elongated peripheral segments of said center section for accommodating elongated pliable patient engaging elements and retaining the patient on the carrier; and an elongated second panel mounted in overlying relation on said center section and adapted to supportingly engage the accommodated patient.

12.   The carrier of claim 11 wherein the first panel side sections are foldably connected to the center section for movement between operative and inoperative modes;

when in an operative mode, said side sections extending angularly upwardly from said center section and being adapted to accommodate therebetween the supported patient, and when in an inoperative mode, said side sections being in substantially coplanar relation with said center section.

13. The carrier of claim 11 wherein the second panel is secured in substantially superposed coincident relation with the first panel center section.

14. The carrier of claim 11 wherein both the first and second panels are formed of triple wall corrugated fibreboard sheet material; the corrugations of each panel extending substantially longitudinally thereof.

15. The carrier of claims 1 and 11 wherein the sheet is a moisture resistant triple wall corrugated fibreboard material.

FIG. 1

21e
21c
20
21
22
21e
21b

FIG. 2

21
21c
21e
21d
X
21f
21a
21f
21e
21d
21b
21e

FIG. 3

30
31d
31a
N        N
31c
31b
31
31c
31c

FIG. 4

T
P
20
21c
21e
21b
T

FIG. 5

P
T
20
21b
22
21a

FIG. 6

FIG. 7

FIG. 9

FIG. 8

FIG. 10